# FASCICULE DE BREVET EUROPEEN

(11) **EP 2 771 290 B1**
(45) Date de publication et mention de la délivrance du brevet: **04.12.2019**
(21) Numéro de dépôt: 12794374.4
(22) Date de dépôt: 25.10.2012
(51) Int. Cl.: C02F 3/28, C05F 17/00, C02F 11/16, C12M 1/107, C05F 17/02, C02F 3/04

(54) **PROCÉDÉ DE VALORISATION DE DÉCHETS**
VERFAHREN ZUR AUFWERTUNG VOM MÜLL
METHOD FOR UPGRADING WASTE

(30) Priorité: 27.10.2011 FR 1159746
(43) Date de publication de la demande: 03.09.2014
(73) Titulaire: VEOLIA PROPRETE, 75008 Paris (FR)
(72) Inventeur: KALLASSY, Monique, 92150 Suresnes (FR); POITRENAUD, Maelenn, 95280 Jouy Le Moutier (FR); CACHO RIVERO, Jesùs Andrés, 78200 Mantes La Jolie (FR); EFREMENKO, Boris, 91320 Wissous (FR)
(74) Mandataire: Novagraaf Technologies
(86) Numéro de dépôt international: PCT/FR2012/052458
(87) Numéro de publication internationale: WO 2013/060992

(56) Documents cités:
- WO-A1-2006/089766
- WO-A2-2011/080766
- DE-A1- 4 409 487
- JP-A- 11 104 604

## Description

La présente invention concerne le domaine du traitement des substrats, en particulier en vue de leur valorisation.

Par « substrat(s) », on entend indistinctement tout type de déchets : déchets ménagers, verts, ou industriels, ou la biomasse. Les substrats se présentent la plupart du temps sous forme en partie solide. Ils comprennent des éléments organiques et éventuellement des éléments inorganiques.

Plus précisément, l'invention concerne selon un premier de ses objets, un procédé de valorisation de substrats au moins en partie organiques et au moins en partie solides, comprenant des étapes consistant à :
A. introduire une pluralité de volumes de substrats frais dans une pluralité de percolateurs respectifs,
B. humidifier le volume de substrats d'au moins un percolateur, pour assurer une hydrolyse d'une partie au moins des constituants organiques dudit volume,
C. injecter le lixiviat issu d'au moins un volume de substrats humidifié dans un digesteur anaérobie produisant du biogaz,
D. renvoyer une partie du liquide issu du digesteur anaérobie dans au moins un percolateur pour l'étape B, et
E. répéter les étapes B à D pendant une durée déterminée les substrats étant laissés en l'état dans le percolateur, sans broyage ni agitation mécanique préalable ou dans le percolateur.

Un tel procédé est connu de l'homme du métier.

Toutefois, les substrats (notamment ménagers) nécessitent généralement en amont de l'humidification une complexe et coûteuse étape de préparation mécanique, ce qui réduit fortement la part de faction organique qui est envoyée en digestion et qui de plus peut poser d'importantes contraintes d'exploitation et de maintenance, dues notamment à la présence d'inertes.

De plus, les substrats (biomasse, déchets verts, papiers ou cartons) contiennent une fraction importante de matière organique non dégradée lors de la digestion anaérobie, leur traitement par digestion entraine donc généralement un surdimensionnement des digesteurs.

Par ailleurs, dans l'art antérieur, il est prévu une étape de percolation monophase dans laquelle les étapes A et B sont mises en œuvre dans une enceinte fermée et étanche, de sorte à produire du biogaz et canaliser le biogaz produit.

L'état de la technique pertinent pour l'objet de la présente invention est représenté par les documents JP 11 104 604, WO 2006/089766, WO 2011/080766 et DE 44 09 487.

La présente invention propose un procédé essentiellement caractérisé en ce qu'il comprend en outre :
- une étape d'analyse chimique du lixiviat utilisé pour l'étape B d'humidification, qui permet de donner une indication sur le potentiel méthanogène d'un volume de substrat, et
- une étape de mesure de la température des substrats dans ledit percolateur,
   et en ce que au-delà d'une valeur de temps seuil, pour un volume de substrats de l'un au moins des percolateurs, ledit procédé comprend en outre des étapes consistant à :
   F. une étape de fermentation aérobie consistant à assurer une première phase de compostage dudit volume de substrats dans ledit percolateur par fermentation aérobie, mise en œuvre en arrêtant d'humidifier le percolateur et en aérant ledit percolateur, et
   G. une étape de maturation aérobie consistant à assurer une deuxième phase de compostage dudit volume de substrats par maturation aérobie, en retirant ledit volume de substrats fermenté dudit percolateur et en positionnant ledit volume dans un dispositif de stockage aérobie pour obtenir un compost stabilisé, dont le taux d'humidité est inférieur à une valeur seuil maximale et/ou supérieur à une valeur seuil minimale, et
   en ce que, pour l'étape C, tout le lixiviat issu dudit volume de substrats humidifié étant injecté directement dans le digesteur anaérobie,
   l'étape F étant asservie à l'étape d'analyse chimique et étant mise en œuvre en fonction du résultat de cette étape d'analyse chimique du lixiviat, par exemple en fonction de l'écart entre une valeur seuil et la valeur absolue de la différence entre une valeur de référence et la valeur du résultat de l'étape d'analyse chimique du lixiviat,
   ladite étape F (181) se terminant lorsque la température des substrats dans ledit percolateur (20) est supérieure à une valeur de température seuil, et
   la mise en œuvre de l'étape G (182) étant asservie aux résultats de l'étape de mesure de la température au moins pendant l'étape de fermentation aérobie (181) et commençant après la réalisation de l'étape E et F.

Par exemple le taux d'humidité est compris entre une valeur seuil minimale égale à 25% et une valeur seuil maximale égale à 70%, un taux supérieur à 25% permettant d'éviter la formation de poussières lors de l'épandage. Les valeurs seuil maximale et/ou minimale du taux d'humidité peuvent par exemple correspondre à une norme ou une réglementation en vigueur.

Avantageusement le procédé comprend en outre des étapes consistant à :
H. extraire tout ou partie du liquide issu du digesteur, et
I. oxygéner tout ou partie du liquide extrait avant son renvoi dans au moins un percolateur pour l'étape B.

Ce qui permet notamment de limiter la présence de bactéries méthanogènes dans un percolateur.

On peut prévoir en outre une étape consistant à mélanger le lixiviat dans le digesteur anaérobie avec d'autres substrats organiques liquides ou pâteux provenant d'autres sources à potentiel méthanogène.

Dans un mode de réalisation, l'un au moins des volumes de substrats frais comprend des éléments inorganiques, le procédé comprenant en outre une étape de séparation, ultérieure à la deuxième phase de compostage, pour séparer les éléments organiques des éléments inorganiques par différentes techniques connues de séparation.

Par rapport aux technologies traditionnelles de traitement de substrats organiques contenant des éléments inorganiques dans lesquels l'étape de séparation s'effectue en amont de l'introduction d'une pluralité de volumes de substrats frais dans une pluralité de percolateurs respectifs, ceci permet de limiter la manutention des substrats et optimise les coûts de traitement.

Avantageusement, on peut prévoir une étape consistant à introduire un nouveau volume de substrats frais dans le percolateur dont le volume de substrats a été retiré pour l'étape de maturation aérobie.

Ce qui permet de pouvoir obtenir une production de méthane constante dans le digesteur, en remplaçant séquentiellement le volume de substrats des percolateurs.

On peut prévoir une étape préalable à l'étape d'introduction et consistant à rendre la matière organique accessible au liquide utilisé pour l'étape d'humidification.

Dans un mode de réalisation, on prévoit en outre une étape de détection de méthane et/ou de sulfure d'hydrogène dans un percolateur, éventuellement couplée à l'étape d'aération dudit percolateur lors de la première phase de compostage.

Le procédé selon l'invention peut être mis en œuvre dans un dispositif comprenant :
- une pluralité de percolateurs dans lesquels une pluralité de volumes de substrats frais respectifs peuvent être introduits,
- des pulvérisateurs pour humidifier le volume de substrats d'au moins un percolateur, et assurer une hydrolyse d'une partie au moins des constituants organiques dudit volume,
- un digesteur anaérobie pour produire du biogaz,
- au moins une pompe pour injecter le lixiviat issu d'au moins un volume de substrats humidifié dans le digesteur, et
- au moins une pompe pour renvoyer une partie du liquide issu du digesteur dans au moins un percolateur.

Le dispositif est essentiellement caractérisé en ce qu'il comprend en outre :
- au moins une canalisation par percolateur par laquelle tout le lixiviat issu dudit volume de substrats humidifié est injecté directement dans le digesteur anaérobie,
- un dispositif d'arrêt de l'humidification d'un percolateur,
- au moins un ventilateur pour assurer une première phase de compostage du volume de substrats d'un percolateur par fermentation aérobie, et
- au moins un lieu ou un dispositif de stockage aérobie, distinct d'un percolateur, pour assurer une deuxième phase de compostage dudit volume de substrats par maturation aérobie pour obtenir un compost stabilisé, dont le taux d'humidité est inférieur à une valeur seuil maximale et/ou supérieur à une valeur seuil minimale.

On peut prévoir en outre au moins une canalisation pour extraire une partie du liquide issu du digesteur, et une cuve aérée pour oxygéner ladite partie extraite.

On peut aussi prévoir en outre un dispositif de séparation des éléments organiques et inorganiques du compost stabilisé, dont le taux d'humidité est inférieur à une valeur seuil maximale et/ou supérieur à une valeur seuil minimale.

La présente invention permet de répondre efficacement à la problématique mondiale de réduction de l'enfouissement des substrats.

Grâce à l'invention, il est possible de réduire la part de la fraction organique mise en Installation de Stockage de Déchets Non Dangereux (ISDND).

Grâce à l'invention, il est possible de traiter intégralement, c'est-à-dire sous forme solide et sous forme liquide, des substrats organiques.

L'absence de manipulation des substrats dans un percolateur, voire pendant la phase de maturation aérobie permet de simplifier l'exploitation industrielle et de réduire la mise en œuvre et la maintenance. En outre, cela améliore la santé des travailleurs puisque le procédé selon l'invention génère moins de poussières et de bioaérosols dus aux nombreuses opérations de manutentions de substrats de l'art antérieur.

D'autres caractéristiques et avantages de la présente invention apparaîtront plus clairement à la lecture de la description suivante donnée à titre d'exemple illustratif et non limitatif et faite en référence aux figures annexées dans lesquelles :
- la figure 1 illustre un mode de réalisation du procédé selon l'invention, et
- la figure 2 illustre un mode de réalisation d'un dispositif susceptible de mettre en œuvre le procédé selon l'invention.

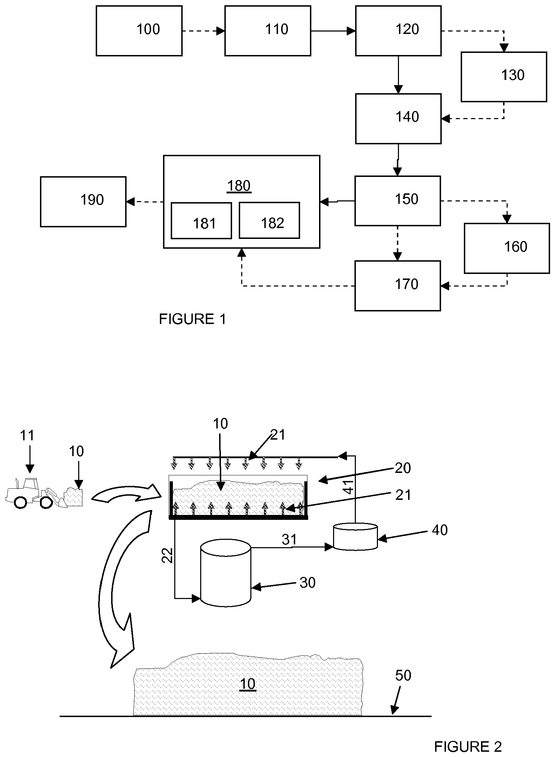

De façon générale, on prévoit que les substrats, en l'espèce des déchets ménagers, soient directement accessibles au liquide permettant l'hydrolyse. Cependant, il arrive que les substrats soient inaccessibles, par exemple parce qu'ils sont enfermés dans un sac plastique. Dans ce cas on prévoit une étape 100 consistant à rendre la matière organique accessible, typiquement par l'ouverture voire de retrait des sacs plastiques, préalablement à l'étape d'introduction dans un percolateur. Ainsi la matière organique peut être humidifiée par le liquide du percolateur, comme décrit ultérieurement. Les pointillés de la figure 1 illustrent l'aspect optionnel.

Il est proposé ici de valoriser les substrats tant sous forme solide (en tant que telle) que sous forme liquide (c'est-à-dire du liquide extrait des substrats sous forme solide).

A cet effet, il est proposé d'introduire 110 une pluralité de volumes de substrats 10 frais en l'état, c'est-à-dire sans agitation mécanique ou broyage de ceux-ci, dans une pluralité de percolateurs 20 respectifs, par exemple par des moyens mécaniques 11 tels qu'un convoyeur, un transporteur ou encore un chargeur. Contrairement aux techniques de l'art antérieur, laisser les substrats frais en l'état permet d'augmenter la vitesse de traitement des substrats pour des raisons exposées ultérieurement. Un volume de substrats est un volume unitaire arbitraire, par exemple un volume déterminé, le volume d'un sac, le volume d'un percolateur, etc.

Par substrats frais, on entend des substrats n'ayant pas subis de transformation chimique industrielle de valorisation majeure préalable, c'est-à-dire par exemple des substrats issus directement d'un centre de collecte.

Un percolateur 20 est une enceinte close étanche à l'eau mais pas aux gaz, elle est équipée de pulvérisateurs 21 permettant d'humidifier ou d'arroser le volume de substrats qu'elle contient et d'assurer ainsi une hydrolyse d'une partie au moins des constituants organiques dudit volume dans une phase d'humidification, ou indistinctement, de percolation.

Entre l'introduction des substrats et l'étape de maturation aérobie, le volume total de liquide pulvérisé sur le volume de substrat lors de l'humidification 120 est de préférence supérieur au volume de substrat dans le percolateur 20.

De préférence, une fois que l'humidification 120 des substrats est mise en œuvre, on ne réintroduit pas de nouveaux substrats dans un percolateur 20 avant la deuxième phase de compostage, dite de maturation aérobie, décrite ultérieurement.

Dans un percolateur, le liquide pulvérisé sur le volume de substrats (autrement appelé massif de substrats) pour l'étape d'humidification (autrement appelée percolation ou arrosage) 120 s'écoule par gravité et se charge de matières organiques au contact des substrats 10. Cette étape de percolation permet une étape d'extraction, en récupérant le liquide en sortie sous forme de lixiviat (autrement appelé percolât) dans une canalisation 22, grâce à des pores situés dans le fond et/ou sur les côtés du percolateur 20.

Comme les substrats ont été laissés en l'état dans le percolateur, sans broyage ni agitation mécanique préalable ou dans le percolateur, il existe naturellement des poches aérobies au sein du massif de substrats.

En outre, la structure, la densité et la granulométrie du massif permettent de faciliter l'écoulement du liquide pulvérisé au sein du massif, ce qui améliore les conditions d'hydrolyse et limite les risques de production de biogaz.

Malgré les conditions aérobies du percolateur, il se peut que des gaz indésirables (CH4, H2S) soient créés. On peut donc prévoir en outre une étape 130 de détection de méthane et/ou de disulfure d'hydrogène dans un percolateur, et des moyens d'aération(non illustrés) pour éliminer ces gaz le cas échéant, par exemple un ventilateur permettant de ventiler les gaz indésirables dans une canalisation spécifique (non illustrée) pour éviter que les gaz indésirables s'échappent du percolateur 20 dans l'atmosphère. En effet, les contraintes réglementaires imposent de valoriser ou de brûler le biogaz.

Le lixiviat issu d'un volume de substrats humidifié est injecté 140, par exemple grâce à au moins une pompe non illustrée, de la canalisation 22 dans un digesteur 30 anaérobie, qui est un équipement distinct des percolateurs 20, étanche aux liquides et aux gaz. Le digesteur anaérobie 30 permet de produire du biogaz à partir du lixiviat dans une phase de digestion anaérobie. Avantageusement, le digesteur anaérobie comprend d'autres substrats organiques liquides ou pâteux provenant d'autres sources et à potentiel méthanogène, par exemple des boues produites par les usines de traitement d'effluents municipaux ou industriels, avec lesquels le lixiviat est mélangé, ce qui permet par exemple d'optimiser l'utilisation d'un digesteur sur un site en utilisant un même digesteur pour plusieurs percolateurs et/ou plusieurs procédés de valorisation de substrats, par exemple des boues d'épuration.

L'humidification dans les percolateurs (dont un seul est illustré en figure 1) permet donc l'hydrolyse de la matière organique contenue dans les substrats et l'extraction de celle-ci par voie liquide pour la production de biogaz dans le digesteur anaérobie 30.

Dans un percolateur, l'étape d'humidification 120 est de préférence pilotée, par exemple via un calculateur pilotant le débit des pulvérisateurs 21. On peut prévoir d'humidifier de façon discontinue, par exemple selon un profil d'humidification, c'est-à-dire une quantité de liquide donnée, dépendant par exemple du volume de substrats, éventuellement de leur épaisseur, et du temps, en référence à un modèle de référence, par exemple stocké dans une mémoire et accessible au calculateur.

Grâce au liquide injecté, l'étape d'humidification 120 permet de maintenir une humidité adéquate pour l'hydrolyse de la matière organique, c'est-à-dire permet la solubilisation et la transformation de macromolécules organiques en acides gras volatiles (AGV). Elle permet également d'entrainer par solubilisation les AGV et les autres molécules organiques solubles, c'est-à-dire de transporter les matières organiques dissoutes vers le digesteur 30.

Tout le lixiviat des différents percolateurs est envoyé directement vers le digesteur anaérobie 30, par au moins une canalisation 22 par percolateur. C'est-à-dire que contrairement aux solutions de l'art antérieur, il n'existe pas de recirculation directe du lixiviat en entrée d'un percolateur : le lixiviat est nécessairement traité par le digesteur 30 avant une recirculation éventuelle. Ceci permet d'optimiser la production de biogaz dans le digesteur.

De préférence, on prévoit que le liquide issu du digesteur en partie renvoyé dans au moins un percolateur est le surnageant du digesteur. Ainsi, on peut prévoir d'extraire 150, par au moins une canalisation 31 et au moins une pompe (non illustrée), tout ou partie du surnageant du digesteur 30. Dans ce cas, on prévoit de préférence d'oxygéner 160 ladite partie extraite, en l'espèce dans une cuve aérée 40.

De préférence, on prévoit que tout le liquide issu du digesteur est renvoyé dans au moins un percolateur.

On peut donc prévoir une étape de traitement aérobie 160 du surnageant du digesteur anaérobie, ce qui permet l'élimination des bactéries méthanogènes contenues dans celui-ci et la réduction voire l'élimination de la concentration d'azote sous forme ammoniacale dissoutes dans celui-ci, ce qui optimise le potentiel d'extraction du liquide dans les percolateurs lors qu'il y est réinjecté et limite les risques de production de méthane dans un percolateur.

A cet effet, la cuve 40 de traitement du surnageant peut par exemple disposer d'un système d'aération réglable en puissance et en mode opératoire par exemple binaire (on/off).

Le surnageant traité devient alors tout ou partie du liquide extracteur utilisé dans les percolateurs pour l'étape d'humidification dans un cycle de percolation-digestion. En fonction du bilan hydrique d'un percolateur, on peut prévoir de compléter le surnageant traité en ajoutant de l'eau « de process », c'est-à-dire des eaux usées ou d'autres lixiviats.

Une partie au moins du liquide issu du digesteur est donc renvoyée grâce à une canalisation 41 et au moins une pompe (non illustrée) dans au moins un percolateur 20 pour l'étape d'humidification. Les étapes d'humidification, de digestion et de réinjection sont répétées de manière cyclique pendant une durée déterminée, dépendant notamment de la nature des substrats 10.

On peut prévoir un mode mixte dans lequel seule une partie du surnageant du digesteur renvoyé vers le percolateur subit une oxygénation ; l'autre partie du surnageant étant mélangée avec la partie oxygénée pour l'étape d'humidification.

On prévoit en outre une étape d'analyse chimique 170 du lixiviat et/ou du liquide utilisé pour l'étape d'humidification, en l'espèce comprenant le surnageant. L'analyse chimique du lixiviat et/ou de celle du surnageant, voire la comparaison de l'analyse chimique du lixiviat à celle du surnageant permet de donner une indication sur le potentiel méthanogène d'un volume de substrat.

Par exemple, l'étape d'analyse chimique du lixiviat, comprend une mesure de la Demande Chimique d'Oxygène (DCO), dans la canalisation 22 en sortie du percolateur. La DCO est un paramètre utilisé par exemple pour la mesure de la charge organique d'un substrat solide ou liquide, exprimé comme la quantité d'oxygène nécessaire pour oxyder la matière organique dudit substrat solide ou liquide.

Une fois l'étape d'humidification terminée, on prévoit une étape de fermentation aérobie 181 dans le percolateur 20, ce qui permet une première phase de compostage du volume de substrats.

De préférence, la fermentation aérobie est mise en œuvre en fonction du résultat de l'étape d'analyse chimique ; par exemple en fonction de l'écart entre une valeur seuil et la valeur absolue de la différence entre une valeur de référence et la valeur du résultat de l'étape d'analyse chimique 170 du lixiviat.

La fermentation aérobie est mise en œuvre en arrêtant d'humidifier le percolateur. Par exemple grâce à un dispositif d'arrêt tel qu'une vanne ou une pompe commandée disposé sur la canalisation reliant la sortie du digesteur, en l'espèce sur la canalisation 41 reliant la sortie de la cuve aérée à l'entrée du percolateur. La fermentation aérobie permet d'éliminer les germes pathogènes des substrats dans le percolateur et de produire un compost non mature.

Le niveau de maturité du compost correspond au niveau de stabilité de sa matière organique, il est donc fonction à la fois du type de substrat composté et du procédé appliqué. Le niveau de maturité peut être exprimé par différents indicateurs, le plus courant étant le Rottegrad, noté de I à V, un compost frais ou non mature correspondant à un Rottegrad II à III, décrit par exemple à l'URL suivante http://wiki.laboratoirelca.com/index.php/Test_de_maturit%C3%A9 . En fonction des utilisations des composts, différents niveaux de maturité peuvent être recherchés.

On entend indistinctement un compost mûr ou mature.

En outre, pour augmenter la vitesse de fermentation, on prévoit une étape d'aération mécanique du percolateur 20. Par exemple on prévoit au moins un ventilateur (non illustré). La ventilation permet en outre d'éliminer d'éventuelles traces de méthane ou de disulfure d'hydrogène.

Comme les substrats ont été laissés en l'état, sans broyage ni agitation mécanique, il existe naturellement des poches aérobies qui facilitent l'écoulement de l'air au sein du massif et la fermentation aérobie.

La fermentation aérobie ayant lieu dans la même enceinte que l'étape d'humidification, la manutention des substrats organiques solides est réduite en l'espèce à néant puisqu'il n'est pas nécessaire de remuer un volume de substrats pendant le cycle de percolation-digestion-réinjection, ni pendant la fermentation aérobie, ni entre ces deux étapes, ce qui permet notamment de gagner du temps. En outre, l'absence d'action mécanique améliore la qualité du compost produit et permet en l'espèce de produire un compost de haute qualité.

En effet, les contaminants inertes indésirables tels que plastiques, verres, potentiellement initialement présents dans les substrats traités sont plus facilement et efficacement éliminés par les outils de tri, postérieurs au traitement biologique car ils n'ont pas été fragmentés. Ceci permet d'atteindre dans les composts finis des niveaux d'indésirables inertes très faibles, tels que exigés par la règlementation (par exemple, pour la règlementation française la norme d'application NFU 44 051, au sujet de laquelle on peut trouver des éléments sur les valeurs limites en inertes et impuretés à l'URL suivante http://wiki.laboratoirelca.com/index.php/NF_U_44-051).

Une fois l'étape de fermentation aérobie considérée comme finie, par exemple lorsque que l'on considère que la matière organique extractible du volume de substrats de l'un au moins des percolateurs est épuisée, le cycle de percolation-digestion-réinjection est arrêté, et commence alors une deuxième phase de compostage du volume de substrats, assurée par maturation aérobie 182.

L'objectif de la maturation aérobie ou stabilisation aérobie 182 est de stabiliser la matière organique restante pour permettre sa valorisation agronomique ultérieure sous forme de compost.

On prévoit une étape de mesure de la température du substrat dans un percolateur. Au-delà d'une valeur de température seuil, et/ou au-delà d'une valeur de temps seuil, on peut considérer l'étape de fermentation aérobie comme finie et mettre en œuvre l'étape de maturation aérobie 182.

Par exemple, l'évolution dans le temps de la température des substrats dans un percolateur passe par une valeur maximale, typiquement 70°C. De préférence, on met en œuvre l'étape de maturation aérobie 182 une fois la valeur maximale de température atteinte.

Pour la maturation aérobie 182, le volume de substrats solides 10 n'a pas besoin de rester dans le percolateur 20. Le volume de substrats fermenté est alors avantageusement enlevé du percolateur 20 et déplacé (symbolisé par la flèche pleine figure 2) dans un autre lieu aérobie, en l'espèce un dispositif de stockage aérobie tel qu'un andain à l'air libre sur une surface 50 en béton, pour obtenir un compost stabilisé, dont le taux d'humidité est inférieur à une valeur seuil maximale et/ou supérieur à une valeur seuil minimale. Avantageusement, le volume utile de l'andain est supérieur à celui d'un percolateur, ainsi un andain peut cumuler des volumes de substrats issus d'une pluralité de percolateurs. L'utilisation d'air libre dans un andain permet également de ne pas avoir obligatoirement de structure (donc de coûts) d'injection d'air ou d'oxygène.

On peut prévoir un système de collecte des potentiels lixiviats issus de l'andain qui constituent une partie de l'eau « de process » évoquée précédemment, et d'évacuation de ceux-ci vers un percolateur ou vers le digesteur.

La phase de maturation aérobie 182 permet la stabilisation complète de la matière organique pour une valorisation ultérieure sous forme de compost. Le volume de substrats reste donc pendant une durée déterminée dans ce lieu aérobie 50. Cette étape de maturation aérobie permet d'obtenir un compost stabilisé, compatible avec une valorisation agronomique de celui-ci, en particulier lorsqu'elle est couplée à une étape de séparation 190 permettant de séparer les éléments organiques des éléments inorganiques.

Le compostage 180 des substrats est réalisé en deux phases : une phase de fermentation aérobie 181 et une phase de maturation aérobie 182.

Le percolateur 20 libéré de son volume de substrats 10 peut être rempli par un nouveau volume de substrats frais pour un nouveau cycle de percolation-digestion-réinjection.

Globalement, un volume de substrats 10 reste une durée déterminée dans un percolateur 20 pour un cycle de percolation-digestion-réinjection, typiquement quelques jours. Le volume de substrats 10 subit alors dans un percolateur une phase de fermentation aérobie 181 pendant une durée déterminée, typiquement quelques semaines. Le volume de substrats est alors retiré du percolateur 20 et placé dans un autre lieu 50 en maturation aérobie 182 pendant une durée déterminée, typiquement quelques mois.

Dans certains cas, l'un au moins des volumes de substrats comprend en outre des éléments inorganiques, qui ne sont pas valorisables sous forme de biogaz ni sous forme de compost.

On prévoit en outre une étape de séparation 190, ultérieure à la deuxième phase de compostage, pour séparer les éléments organiques des éléments inorganiques. On utilise à cet effet des moyens de séparation (non illustrés) par exemple des moyens de séparation granulométrique, des moyens aérauliques, des moyens optiques, densimétriques ou autres.

Avantageusement, grâce à une séparation en aval, les substrats ne sont ni broyés, ni remués, ni criblés en amont de la chaine de traitement, ce qui permet une élimination beaucoup plus aisée et moins chère des éléments inorganiques indésirables, ce qui permet notamment d'éviter la présence d'inertes (par exemple le verre) dans le compost. La séparation sur un compost aval plus sec que les substrats frais implique une réduction très importante du coût de l'étape de séparation, notamment parce que la masse à traitée est significativement réduite et que la viscosité est moindre, donc la performance des équipements de séparation augmente significativement.

Par ailleurs, on notera que l'étape de séparation 190 implique une fourniture d'énergie aux moyens mécaniques de séparation. Or à ce stade, les substrats organiques ont été partiellement valorisés sous forme liquide grâce au digesteur. La production de méthane de celui-ci peut donc avantageusement permettre la fourniture en énergie auxdits moyens mécaniques de séparation.

L'étape de séparation 190 permet également d'obtenir un matériel non-organique de meilleure qualité puisqu'exempt de matériel organique. Le matériel inorganique peut être valorisé par exemple comme Combustible Solide de Récupération (CSR), ou dans différentes utilisations de recyclage ou de stockage.

Grâce à l'invention, les substrats solides sont laissés au repos : ils ne sont pas remués tant qu'ils sont dans un percolateur, et peuvent ne pas être remués non plus pendant l'étape de maturation aérobie, ce qui permet d'obtenir un compost de haute qualité sans bris d'inertes.

Grâce à l'invention, l'étape d'humidification peut durer au maximum trois semaines et l'étape de fermentation aérobie peut durer au maximum six semaines. Par conséquent, un volume de substrat reste dans un percolateur au maximum deux mois.

Ce traitement très rapide en moins de deux mois a comme avantages :
- de pouvoir augmenter la production d'un site de traitement,
- de diminuer la surface nécessaire au stockage des substrats frais, et
- de permettre en outre la production d'un compost de haute qualité.

Avantageusement, le procédé peut être exempt de tout traitement thermique du lixiviat, ce qui permet notamment un gain de coût et de place.

On notera que les substrats restent solides dans le percolateur pour l'humidification comme pour la fermentation aérobie, ils ne sont pas pâteux et ne peuvent pas être pompés.

## Revendications

1. Procédé de valorisation de biomasse ou de substrats au moins en partie organiques et au moins en partie solides, comprenant des étapes consistant à :
A. introduire (110) une pluralité de volumes de substrats (10) frais dans une pluralité de percolateurs (20) respectifs,
B. humidifier (120) le volume de substrats d'au moins un percolateur (20), pour assurer une hydrolyse d'une partie au moins des constituants organiques dudit volume,
C. injecter (140) le lixiviat issu d'au moins un volume de substrats humidifié dans un digesteur anaérobie (30) pour produire du biogaz,
D. renvoyer une partie au moins du liquide issu du digesteur anaérobie (30) dans au moins un percolateur (20) pour l'étape B (120), et
E. répéter les étapes B à D pendant une durée déterminée, les substrats étant laissés en l'état dans le percolateur (20), sans broyage ni agitation mécanique préalable ou dans le percolateur (20),
ledit procédé étant **caractérisé en ce qu'**il comprend en outre:
- une étape (170) d'analyse chimique du lixiviat utilisé pour l'étape B d'humidification, qui permet de donner une indication sur le potentiel méthanogène d'un volume de substrat, et
- une étape de mesure de la température des substrats dans ledit percolateur (20),
et **en ce que** au-delà d'une valeur de temps seuil, pour un volume de substrats de l'un au moins desdits percolateurs, ledit procédé comprend en outre des étapes consistant à :,
F. une étape de fermentation aérobie (181) consistant à assurer une première phase de compostage dudit volume de substrats dans ledit percolateur (20) par fermentation aérobie (181) mise en œuvre en arrêtant d'humidifier le percolateur (20) et en aérant ledit percolateur (20), et
G. une étape de maturation aérobie (182) consistant à assurer une deuxième phase de compostage dudit volume de substrats par maturation aérobie (182), en retirant ledit volume de substrats fermenté dudit percolateur (20) et en positionnant ledit volume dans un dispositif de stockage aérobie (50) pour obtenir un compost stabilisé, dont le taux d'humidité est inférieur à une valeur seuil maximale et/ou supérieur à une valeur seuil minimale, et
**en ce que**, pour l'étape C, tout le lixiviat issu dudit volume de substrats humidifié étant injecté directement dans le digesteur anaérobie (30),
l'étape F (181) étant asservie à l'étape (170) d'analyse chimique et étant mise en œuvre en fonction du résultat de cette étape d'analyse chimique du lixiviat, par exemple en fonction de l'écart entre une valeur seuil et la valeur absolue de la différence entre une valeur de référence et la valeur du résultat de l'étape (170) d'analyse chimique du lixiviat,
ladite étape F (181) se terminant lorsque la température des substrats dans ledit percolateur (20) est supérieure à une valeur de température seuil, et
la mise en œuvre de l'étape G (182) étant asservie aux résultats de l'étape de mesure de la température au moins pendant l'étape de fermentation aérobie (181) et commençant après la réalisation de l'étape E et F.

2. Procédé selon la revendication 1, comprenant en outre des étapes consistant à :
H. extraire (150) tout ou partie du liquide issu du digesteur, et
I. oxygéner (160) tout ou partie du liquide extrait avant son renvoi dans au moins un percolateur pour l'étape B.

3. Procédé selon l'une quelconque des revendications précédentes, comprenant en outre une étape consistant à mélanger le lixiviat dans le digesteur anaérobie avec d'autres substrats organiques liquides ou pâteux provenant d'autres sources à potentiel méthanogène.

4. Procédé selon l'une quelconque des revendications précédentes, dans lequel l'un au moins des volumes de substrats frais comprend des éléments inorganiques, le procédé comprenant en outre une étape, ultérieure à la deuxième phase de compostage (182), de séparation (190) des éléments organiques et inorganiques.

5. Procédé selon l'une quelconque des revendications précédentes, comprenant une étape consistant à introduire un nouveau volume de substrats frais dans le percolateur (20) dont le volume de substrats a été retiré pour l'étape de maturation aérobie (182).

6. Procédé selon l'une quelconque des revendications précédentes, comprenant une étape (100) préalable à l'étape d'introduction et consistant à rendre la matière organique accessible au liquide utilisé pour l'étape d'humidification.

7. Procédé selon l'une quelconque des revendications précédentes, comprenant en outre une étape (130) de détection de méthane et/ou de sulfure d'hydrogène dans un percolateur (20), éventuellement couplée à l'étape d'aération dudit percolateur lors de la première phase de compostage.

## Patentansprüche

1. Verfahren zur Aufwertung vom Biomasse oder von Substraten, die mindestens teilweise organisch und mindestens teilweise fest sind, umfassend folgende Schritte:
A. Einführen (110) einer Vielzahl von Volumina von frischen Substraten (10) in eine Vielzahl von entsprechenden Perkolatoren (20),
B. Befeuchten (120) des Volumens von Substraten von mindestens einem Perkolator (20), um eine Hydrolyse mindestens eines Teils der organischen Bestandteile des Volumens sicherzustellen,
C. Injizieren (140) des Sickerwassers, das aus mindestens einem befeuchteten Volumen von Substraten stammt, in einen anaeroben Faulbehälter (30), um Biogas zu erzeugen,
D. Zurückschicken mindestens eines Teils der Flüssigkeit, die aus dem anaeroben Faulbehälter (30) stammt, in mindestens einen Perkolator (20) für den Schritt B (120), und
E. Wiederholen der Schritte B bis D während einer bestimmten Dauer, wobei die Substrate in ihrem Zustand im Perkolator (20) gelassen werden, ohne vorheriges oder im Perkolator (20) durchgeführtes Malen oder mechanisches Rühren,
wobei das Verfahren **dadurch gekennzeichnet ist, dass** es außerdem Folgendes umfasst:
- einen Schritt (170) des chemischen Analysierens des Sickerwassers, das für den Schritt B des Befeuchtens verwendet wird, der ermöglicht, eine Anzeige über das methanogene Potenzial eines Substratvolumens anzugeben, und
- einen Schritt des Messens der Temperatur der Substrate im dem Perkolator (20),
und, dadurch, dass über einen Schwellenzeitwert hinaus für ein Volumen von Substraten des mindestens einen der Perkolatoren das Verfahren außerdem folgende Schritte umfasst:
F. einen Schritt des aeroben Fermentierens (181), der darin besteht, eine erste Phase der Kompostierung des Volumens von Substraten in dem Perkolator (20) durch aerobes Fermentieren (181) sicherzustellen, die durchgeführt wird, indem das Befeuchten des Perkolators (20) gestoppt und der Perkolator (20) belüftet wird, und
G. einen Schritt des aeroben Reifens (182), der darin besteht, eine zweite Phase der Kompostierung des Volumens von Substraten durch aerobe Reifung (182) sicherzustellen, indem das fermentierte Volumen von Substraten vom Perkolator (20) entnommen wird, und indem das Volumen in eine aerobe Speichervorrichtung (50) positioniert wird, um einen stabilisierten Kompost zu erhalten, dessen Feuchtigkeitsgehalt geringer als ein maximaler Schwellenwert und/oder höher als ein minimaler Grenzwert ist, und
dadurch, dass bei Schritt C das gesamte Sickerwasser, das aus dem befeuchteten Volumen von Substraten stammt, direkt in den anaeroben Faulbehälter (30) injiziert wird,
wobei Schritt F (181) vom Schritt (170) des chemischen Analysierens abhängt und je nach dem Ergebnis dieses Schritts des chemischen Analysierens des Sickerwassers durchgeführt wird, z. B. je nach dem Abstand zwischen einem Schwellenwert und dem absoluten Wert der Differenz zwischen einem Referenzwert und dem Wert des Ergebnisses des Schritts (170) des chemischen Analysierens des Sickerwassers,
wobei der Schritt F (181) endet, wenn die Temperatur der Substrate im dem Perkolator (20) höher als ein Schwellentemperaturwert ist und
wobei die Durchführung von Schritt G (182) von den Ergebnissen des Schritts des Messens der Temperatur mindestens während des Schritts des aeroben Fermentierens (181) abhängt und nach der Durchführung von Schritt E und F beginnt.

2. Verfahren nach Anspruch 1, umfassend außerdem folgende Schritte:
H. Extrahieren (150) der Gesamtheit oder eines Teils der Flüssigkeit, die aus dem Faulbehälter stammt, und
I. Belüften (160) der Gesamtheit oder eines Teils der Flüssigkeit, die vor ihrem Zurückschicken in mindestens einen Perkolator für Schritt B extrahiert wurde.

3. Verfahren nach einem der vorhergehenden Ansprüche, außerdem umfassend einen Schritt, der darin besteht, das Sickerwasser im aeroben Faulbehälter mit anderen flüssigen oder pastösen organischen Substraten zu mischen, die aus anderen Quellen mit methanogenem Potenzial stammen.

4. Verfahren nach einem der vorhergehenden Ansprüche, wobei mindestens eines der Volumina von frischen Substraten anorganische Elemente umfasst, wobei das Verfahren außerdem einen Schritt nach der zweiten Phase der Kompostierung (182) des Trennens (190) der organischen und anorganischen Elemente umfasst.

5. Verfahren nach einem der vorhergehenden Ansprüche, umfassend einen Schritt, der darin besteht, ein neues Volumen von frischen Substraten in den Perkolator (20) einzuführen, dessen Volumen von Substraten für den Schritt des aeroben Reifens (182) entnommen wurde.

6. Verfahren nach einem der vorhergehenden Ansprüche, umfassend einen Schritt (100) vor dem Schritt des Einführens, der darin besteht, die organische Materie für die Flüssigkeit zugänglich zu machen, die für den Schritt des Befeuchtens verwendet wird.

7. Verfahren nach einem der vorhergehenden Ansprüche, umfassend außerdem einen Schritt (130) des Nachweisens von Methan und/oder Schwefelwasserstoff in einem Perkolator (20), eventuell gekoppelt mit dem Schritt des Belüftens des Perkolators während der ersten Phase der Kompostierung.

## Claims

1. Method for upgrading biomass or substrates at least partially organic and at least partially solid, comprising steps consisting in:
A. introducing (110) a plurality of volumes of fresh substrates (10) in a plurality of respective strainers (20),
B. humidifying (120) the volume of substrates of at least one strainer (20), in order to ensure a hydrolysis of at least a portion of the organic constituents of said volume,
C. injecting (140) the leachate from at least one humidified volume of substrates into an anaerobic digester (30) in order to produce biogas,
D. sending at least a portion of the liquid from the anaerobic digester (30) into at least one strainer (20) for the step B (120), and
E. repeating the steps B to D for a determined duration, with the substrates being left as is in the strainer (20), without crushing or mechanical stirring beforehand or in the strainer (20),
said method being **characterised in that** it further comprises:
- a step (170) of chemically analysing the leachate used for the step B of humidification, which makes it possible to provide an indication on the methanogenic potential of a volume of substrate, and
- a step of measuring the temperature of the substrates in said strainer (20),
and **in that** beyond a threshold time value, for a volume of substrates of at least one of said strainers, said method further comprises steps consisting in:
F. a step of aerobic fermentation (181) consisting in providing a first phase of composting of said volume of substrates in said strainer (20) via aerobic fermentation (181) implemented by stopping the humidification of the strainer (20) and by aerating said strainer (20), and
G. a step of aerobic maturation (182) consisting in providing a second phase of composting of said volume of substrates par aerobic maturation (182), by withdrawing said fermented volume of substrates from said strainer (20) and by positioning said volume in an aerobic storage device (50) in order to obtain a stabilised compost, of which the humidity rate is less than a maximum threshold value and/or greater than a minimum threshold value, and
**in that**, for the step C, all the leachate from said humidified volume of substrates being injected directly into the anaerobic digester (30),
the step F (181) being dependent on the step (170) of chemical analysis and being implemented according to the result of this step of chemical analysis of the leachate, for example according to the difference between a threshold value and the absolute value of the difference between a reference value and the value of the result of the step (170) of chemical analysis of the leachate,
said step F (181) ending when the temperature of the substrates in said strainer (20) is greater than a threshold temperature value, and
the implementing of the step G (182) being dependent on the results of the step of measuring the temperature at least during the step of aerobic fermentation (181) and beginning after the realisation of the step E and F.

2. Method according to claim 1, further comprising steps consisting in:
H. extracting (150) all or a portion of the liquid from the digester, and
I. oxygenating (160) all or a portion of the extracted liquid before sending it back into at least one strainer for the step B.

3. Method according to any preceding claim, further comprising a step consisting in mixing the leachate in the anaerobic digester with other liquid or pasty organic liquids coming from other sources with methanogenic potential.

4. Method according to any preceding claim, wherein at least one of the fresh volumes of substrates comprises inorganic elements, with the method further comprising a step, after the second phase of composting (182), of separating (190) organic and inorganic elements.

5. Method according to any preceding claim, comprising a step consisting in introducing a new fresh volume of substrates into the strainer (20) of which the volume of substrates has been removed for the step of aerobic maturation (182).

6. Method according to any preceding claim, comprising a step (100) prior to the step of introducing and consisting in rendering the organic material accessible to the liquid used for the step of humidification.

7. Method according to any preceding claim, further comprising a step (130) of detecting methane and/or hydrogen sulphide in a strainer (20), optionally coupled with the step of aerating said strainer during the first phase of composting.
